(19) Europäisches Patentamt
European Patent Office
Office européen des brevets

(11) Publication number: **0 072 027**
**B1**

(12) # EUROPEAN PATENT SPECIFICATION

(45) Date of publication of patent specification: **18.05.88**

(51) Int. Cl.$^4$: **C 07 D 473/16,**
C 07 D 473/18, A 61 K 31/52

(21) Application number: **82107247.7**

(22) Date of filing: **10.08.82**

(54) **Antiviral compounds.**

(30) Priority: **11.08.81 GB 8124444**

(43) Date of publication of application:
**16.02.83 Bulletin 83/07**

(45) Publication of the grant of the patent:
**18.05.88 Bulletin 88/20**

(84) Designated Contracting States:
**AT BE CH DE FR GB IT LI LU NL SE**

(56) References cited:
**EP-A-0 049 072**
**EP-A-0 066 208**
**EP-A-0 085 424**
**GB-A-1 523 865**

**The file contains technical information submitted after the application was filed and not included in this specification**

(73) Proprietor: **THE WELLCOME FOUNDATION LIMITED**
**183-193 Euston Road**
**London NW1 2BP (GB)**

(72) Inventor: **Schaeffer, Howard John**
**4112 Oak Park Road**
**Raleigh, North Carolina 27612 (US)**

(74) Representative: **Sandmair, Kurt, Dr. et al**
**Patentanwälte Schwabe, Sandmair, Marx**
**Stuntzstrasse 16**
**D-8000 München 80 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 072 027 B1

**Description**

This invention relates to antiviral purine derivatives containing an acyclic side chain in the 9-position. The invention also relates to methods of synthesising these compounds, pharmaceutical and veterinary compositions containing them, and their use in the treatment of viral infections in mammals.

Ogilvie and Gillen, in *Tetrahedron Letters, 21,* 1980, 327—330, describe the synthesis of the bis-(hydroxymethyl)methoxymethyl derivatives of adenine but no biological activity was disclosed. In this reference the compound was tested with adenosine deaminase and found it to be a poor substrate for the enzyme and a weak competitive inhibitor. 9 - [(2 - Hydroxy - 1 - hydroxymethylethoxy)methyl] - 2,6 - diaminopurine is embraced within the general formula of UK Patent Specification No. 1523865 but is not disclosed specifically therein. European patent specification 49072 describes a sub-class of compounds falling within the above-mentioned UK Patent Specification No. 1523865 and including the compound 9 - [(2 - hydroxy - 1 - hydroxymethylethoxy)methyl]guanine. UK Patent Specification No. 1523865 discloses a group of acyclic nucleosides, in particular 9-(2-hydroxyethoxymethyl) derivatives of purines, which have been found to have antiviral activity against various classes of DNA and RNA viruses both *in vitro* and *in vivo*. In particular these compounds possess activity against the herpes group of viruses which includes *H. simplex, H. zoster* and *H. varicella*. These viruses cause such diseases as herpetic keratitis, herpetic encephalitis, cold sores, shingles and genital infections. Of the compounds specifically disclosed in UK Patent Specification No. 1523865, 9-(2-hydroxyethoxymethyl) guanine, otherwise known as acyclovir, has been found to be particularly active against many *H. simplex* infections but suffers from the disadvantage of being poorly soluble in aqueous systems.

It has now been found unexpectedly that the compounds of formula (I) below, in contrast to other compounds disclosed in UK Patent Specification No. 1523865 and heretofore tested, have useful antiviral activity sufficient to treat equine rhinopneumonitis virus *in vivo*. In experiments in mice, the compounds have also been found to have advantageous activity against herpetic encephalitis. Furthermore these compounds have the selective advantage of greater solubility in water compared with acyclovir which improves the possibility of being able to obtain high levels of the drug in the plasma without the risk of renal complications.

According to the present invention there is provided a compound of formula (I)

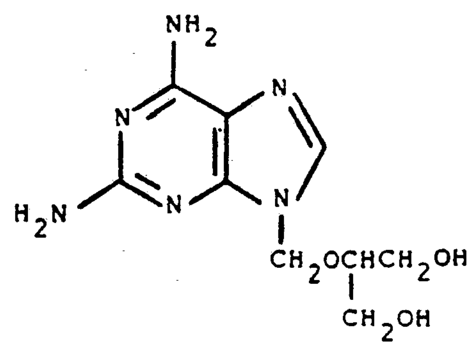

(I)

and physiologically acceptable salts thereof.

As mentioned above, the compounds of formula (I) have the important advantage of having a greater solubility in water than acyclovir.

Salts of the compound of formula (I) which may be conveniently used in therapy include physiologically acceptable salts of organic acids such as lactic, acetic, malic or p-toluenesulphonic acid as well as physiologically acceptable salts of mineral acids such as hydrochloric or sulphuric acid.

The present invention further includes the physiologically acceptable esters of compounds of formula (I'):—

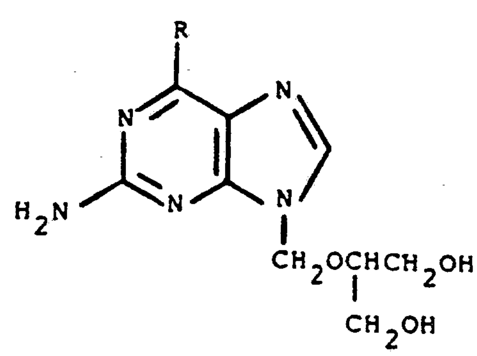

(I')

(wherein R represents a hydroxy or amino group) and wherein the ester is a formyloxy or $C_{1-16}$ (for

example $C_{1-6}$) alkanoyloxy (e.g. acetoxy or propionyloxy), optionally substituted aralkanoyloxy (e.g. phenyl-$C_{1-4}$alkanoyloxy such as phenyl-acetoxy) or optionally substituted aroyloxy (e.g. benzoyloxy or naphoyloxy) ester grouping at one or both of the terminal positions of the 9-side chain of the compounds of formula (I'). The above-mentioned aralkanoyloxy and aroyloxy ester groups may be substituted, for example by one or more halogen (e.g. chlorine or bromine) atoms or amino, nitrile of sulphamido groups, the aryl moiety of the grouping advantageously containing 6 to 10 carbon atoms.

The compounds of formula (I) and their salts may be prepared in conventional manner by analogous processes for preparing compounds of similar structure, such as those methods described in GB—PS 1523865.

In a second aspect of the present invention there is provided a process for preparing compounds of formula (I') and physiologically acceptable salts and esters thereof as defined as hereinbefore, characterized in that

(a) a compound of formula (II)

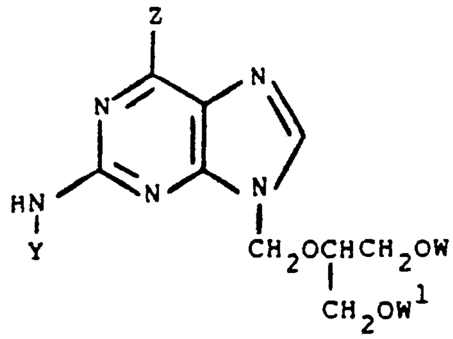

(II)

(wherein W and $W^1$ each represent a hydrogen atom or a blocking group, Y is a hydrogen atom of a blocking group and Z is a group of formula —OY or —NHY, wherein Y is as defined above with the provisos that (a) when Z represents a hydroxy group and Y represents an acyl blocking group, W and $W^1$ each represent a blocking group other than an aralkyl group, (b) when Z represents a hydroxy group and W and $W^1$ each represent a hydrogen atom, Y represents hydrogen or a blocking group other than an acyl blocking group, and (c) at least one of W, $W^1$ and Y represents a blocking group), is deblocked to form a compound of formula (I') or a salt or ester thereof;

(b) a compound of formula (III)

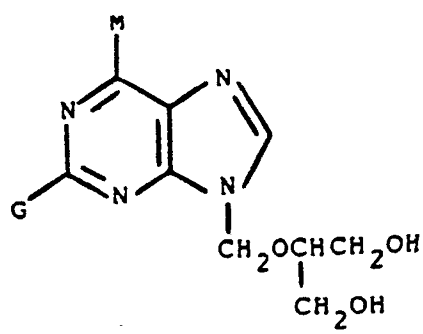

(III)

(wherein M is a 6-hydroxy or amino group and G is an atom or other group than an acyl group that can be replaced by or converted to an amino group, or G is a 2-amino group and M is an atom or group that can be replaced by or converted to an amino or hydroxy group) or a salt or ester thereof is converted to a compound of formula (I') or a salt or ester thereof;

(c) a compound of formula (IV)

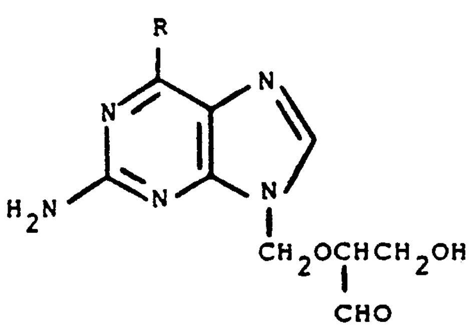

(IV)

(wherein R is as defined above or a salt or ester thereof is reduced;

(d) a compound of the formula

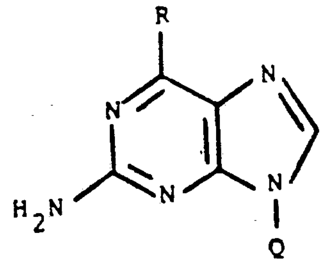

(V)

(wherein R is as defined above and Q is a leaving group or atom) is reacted with a compound of formula

$ACH_2OCHCH_2OH$
|
$CH_2OH$ (VI)

(wherein A is a leaving group or atom);

(e) hydrolysis of a compound of formula

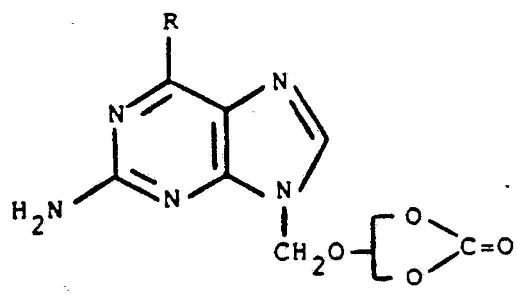

(VI)

(wherein R is as defined above); and optionally effecting one or more of the following conversions, in any desired sequence:

(i) where the resulting product is a base, converting the said base into a physiologically acceptable acid addition salt thereof;

(ii) where the resulting product is an acid addition salt, converting the said salt into the parent base;

(iii) where the resulting product is a compound of formula (I') or a salt thereof converting the said compound or salt thereof into a physiologically acceptable ester of the said compound or salt; and/or

(iv) where the resulting product is an ester of a compound of formula (I), converting the said ester into the parent compound of formula (I') or a physiologically acceptable salt thereof.

In method a) the blocking groups W, $W^1$ and Y may be selected for example from acyl groups such as $C_{1-4}$alkanoyl groups e.g. acetyl, aroyl grops, e.g. benzoyl; arylmethyl groups e.g. benzyl; or tri-$C_{1-4}$alkylsilyl e.g. trimethylsilyl. Arylmethyl blocking groups, may be removed for example by hydrogenolysis, e.g. by hydrogenation in the presence of Raney nickel or a palladium catalyst or by the use of sodium in liquid ammonia. Acyl blocking groups may be removed for example by hydrolysis using for example an amine such as methylamine or triethylamine, advantageously in an aqueous medium. Trialkylsilyl blocking groups may be removed for example by solvolysis e.g. with alcoholic or aqueous ammonia, or by alcoholysis.

Conversion of a compound of formula (III) into a compound of formula (I'), by method b), can be achieved by various means. For example M and/or G may each represent an azide group which can be reduced to an amino group by catalytic hydrogenation using a suitable catalyst such as palladium. Alternatively, M and/or G may each represent a halogen atom or an alkylthio or alkylsulphonyl group which can be converted to an amino group by aminolysis using for example ammonia. For the preparation of the compound of formula (I') wherein R is a hydroxy group, a compound of formula (III) wherein M is an amino group may be converted for example by treatment with nitrous acid. Alternatively, a compund of formula (III) wherein M is a mercapto or alkylthio group may be converted into a compond of formula (I') wherein R is a hydroxy group by oxidation and hydrolysis in conventional manner. Also, a compound of formula (III) wherein M is halogen can be converted into a compound of formula (I') wherein R is hydroxy by treatment with 2-mercaptoethanol and an alkali metal alkoxide, e.g. sodium methoxide.

These processes together with other conventional processes are described in Fused Pyrimidines, Part II, Purines Ed. by D. J. Brown (1971), Wiley-Interscience. In a further alternative a compound of formula (III) wherein M is an amino group may be converted into a compound of formula (I') wherein R is a hydroxy group by treatment with a deaminating enzyme such as adenosine deaminase.

Reduction of a compound of formula (IV) in process (c), may be achieved for example by reaction with an appropriate aldehyde reducing agent such as sodium borohydride, sodium cyanoborohydride, tetraethylammonium borohydride or pyridine/diborane/tetrahydrofuran/trifluoroacetic acid.

In process (d), the group Q in formula (V) may for example represent a hydrogen atom; an acyl group, e.g. a $C_{1-4}$alkanoyl group such as an acetyl group or an aroyl group such as benzoyl group; or a tri-$C_{1-4}$alkylsilyl group such as a trimethylsilyl group. The group A in formula (VI) may for example represent a halogen atom (e.g. chlorine) or an acyloxy group wherein the acyl moiety may be for example a $C_{1-4}$alkanoyl group such as acetyl or an aroyl group such as benzoyl. The reaction may be conveniently effected in a strong polar solvent such as dimethylformamide or hexamethylphosphoramide, advantageously in the presence of a base such as triethylamine or potassium carbonate. Alternatively, a thermal condensation may be effected by heating the compounds of formulae (V) and (VI) in the presence of a catalytic amount of a strong acid, e.g. sulphuric acid.

In process (e), hydrolysis of the compound of formula (VII) may be effected for example under basic conditions, e.g. by treatment with an organic amine such as methylamine or triethylamine.

In a process incorporating processes a) and b) above, a compound of formula (VIII)

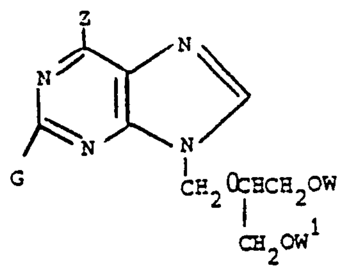

(VIII)

wherein W W[1] and Z are as defined above and G represents a halogen atom can be treated with alcoholic ammonia to give the desired end-product of formula (I').

Compounds of formula (II) to (VIII) employed as intermediates in the synthesis of the compounds of formula (I) can be prepared in conventional manner, e.g. by procedures described in UK Patent Specification No. 1523865. These methods rely on intermediates prepared from simply substituted purines, which may be available commercially, or prepared according to techniques which are well known *per se* and which are disclosed in the literature such as the aforementioned text-book.

Thus, for example, compounds of formulae (II) and (III) may be generally prepared by using an analogous procedure to that of process (d), i.e. reacting an appropriate purine wherein the 2-, 6-, and/or 9-positions are optionally protected, e.g. by acyl or trialkylsilyl groups of the type described above, with a compound of formula (VI) wherein the terminal hydroxy groups are optionally protected by acyl or trialkylsilyl groups of the type described, and subsequently, as necessary and/or desired, removing any of the said protecting groups, prior to use in processes (a) or (b). Alternatively, a compound of formula (II) wherein W and W[1] each represent a benzoyl blocking group can be prepared for example by treatment of a corresponding 9-bis(chloromethyl)-methoxy (or thio) methyl purine analogue with a benzoylating agent, e.g. sodium benzoate the starting purine analogue being prepared for example by treatment of a compound of formula (V), optionally protectd in the 2- and/or 6-positions, with a compound of formula

$$ACH_2OCHCH_2Cl$$
$$|$$
$$CH_2Cl$$

(wherein A is described above).

Compounds of formula (IV) may be prepared in conventional manner, e.g. as herein described in Example 2 of the present invention or by analogous procedures.

The present invention also provides a compound of formula (I) and physiologically acceptable salts thereof and the physiologically acceptable esters of compounds of formula (I') for use in the treatment or prophylaxis of a viral disease in an animal, e.g. a mammal such as man. Such compounds and their salts and esters are hereinafter collectively referred to as the active ingredients.

The compounds are especially useful for the treatment or prophylaxis of diseases causd by various DNA viruses, such as herpes infections for example herpes simplex, varicella or zoster, cytomegalovirus as well as diseases caused by hepatitis B or Epstein-Barr viruses. The compounds can also be used for the treatment or prophylaxis of papilloma or wart virus infections. In addition to their use in human medical therapy, the compounds can be administered to other animals for treatment or prophylaxis of viral diseases, e.g. in other mammals. For example, the compounds of formula (I) and (I') are especially useful for the treatment of equine rhinopneumonitis.

The active ingredients may be administered by any route appropriate to the condition to be treated, suitable routes including oral, rectal, nasal, topical (including buccal and sublingual), vaginal and parenteral (including subcutaneous, intramuscular, intravenous, intradermal, intrathecal and epidural). It

will be appreciated that the preferred route may vary with for example the condition of the recipient.

For each of the above-indicated utilities and indications the amount required of an active ingredient (as above defined) will depend upon a number of factors including the severity of the condition to be treated and the identity of the recipient and will ultimately be at the discretion of the attendant or veterinarian. In general however, for each of these utilities and indications, a suitable, effective dose will be in the range of 0.1 to 250 mg per kilogram bodyweight of recipient per day, preferably in the range of 1 to 100 mg per kilogram bodyweight per day and most preferably in the range of 5 to 20 mg per kilogram bodyweight per day; an optimum dose is about 10 mg per kilogram bodyweight per day. (Unless otherwise indicated all weights of active ingredient are calculated as the parent compound of formula (I) and (I'): for salts and esters thereof the figures would be increased proportionately.) The desired dose is preferably presented as two, three, four or more sub-doses administered at appropriate intervals throughout the day. These sub-doses may be administered in unit dosage forms, for example, containing 10 to 1000 mg, preferably 20 to 500 mg and most preferably 100 to 400 mg of active ingredient per unit dosage form.

While it is possible for the active ingredients to be administered alone it is preferable to present them as pharmaceutical formulations. The formulations, both for veterinary and for human use, of the present invention comprise at least one active ingredient, as above defined, together with one or more acceptable carriers therefor and optionally other therapeutic ingredients. The carrier(s) must be "acceptable" in the sense of being compatible with the other ingredients of the formulation and not deleterious to the recipient thereof.

The formulations include those suitable for oral, rectal, nasal, topical (including buccal and sublingual), vaginal or parenteral (including subcutaneous, intramuscular, intravenous, intradermal, intrathecal and epidural) administration. The formulations may conveniently be presented in unit dosage form and may be prepared by any of the methods well known in the art of pharmacy. Such methods include the step of bringing into association the active ingredient with the carrier which constitutes one or more accessory ingredients. In general the formulations are prepared by uniformly and intimately bringing into association the active ingredient with liquid carriers of finely divided solid carriers or both, and then, if necessary, shaping the product.

Formulations of the present invention suitable for oral adminstration may be presented as discrete units such as capsules, cachets or tablets each containing a predetermined amount of the active ingredient; as a powder or granules; as asolution or a suspension in an aqueous liquid or a non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, preservative, surface active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein.

For infections of the eye or other external tissues, e.g. mouth and skin, the formulations are preferably applied as a topical ointment or cream containing the active ingredient in an amount of, for example, 0.075 to 20% w/w, preferably 0.2 to 15% w/w and most preferably 0.5 to 10% w/w. When formulated in an ointment, the active ingredients may be employed with either paraffinic or a water-miscible ointment base. Alternatively, the active ingredients may be formulated in a cream with an oil-in-water cream base.

If desired, the aqueous phase of the cream base may include, for example, at least 30% w/w of a polyhydric alcohol, i.e. an alcohol having two or more hydroxyl groups such as propylene glycol, butane 1,3-diol, mannitol, sorbitol, glycerol and polyethylene glycol and mixtures thereof. The topical formulations may desirably include a compound which enhances absorption or penetration of the active ingredient through the skin or other affected areas. Examples of such dermal penetration enhancers include dimethylsulphoxide and related analogues.

The oily phase of the emulsions of this invention may be constituted from known ingredients in a known manner. While the phase may comprise merely an emulsifier (otherwise known as an emulgent), it desirably comprises a mixture of at least one emulsifier with a fat or an oil or with both a fat and an oil. Preferably, a hydrophilic emulsifier is included together with a lipophilic emulsifier which acts as a stabiliser. It is also preferred to include both an oil and a fat. Together, the emulsifier(s) with or without stabiliser(s) make up the so-called emulsifying wax, and the wax together with the oil and/fat make up the so-called emulsifying ointment base which forms the oily dispersed phase of the cream formulations.

Emulgents and emulsion stabilisers suitable for use in the formulation of the present invention include Tween® 60, Span® 80, cetostearyl, alcohol, myristyl alcohol, glyceryl mono-stearate and sodium lauryl sulphate.

The choice of suitable oils or fats for the formulation is based on achieving the desired cosmetic properties, since the solubility of the active compound in most oils likely to be used in pharmaceutical emulsion formulations is very low. Thus the cream should preferably be a non-greasy, non-staining and washable product with suitable consistency to avoid leakage from tubes or other containers. Straight or branched chain, mono- or dibasic alkyl esters such as di-isoadipate, isocetyl stearate, propylene glycol

diester of coconut fatty acids, isopropyl myristate, decyloleate, ispropyl palmitate, butyle stearate, 2-ethylhexyl palmitate or a blend of branched chain esters known as Crodamol® CAP may be used, the last three being preferred esters. These may be used alone or in combination depending on the properties required. Alternatively, high melting point lipids such as white soft paraffin and/or liquid paraffin or other mineral oils can be used.

Formulations suitable for topical administration to the eye also include eye drops wherein the active ingredient is dissolved or suspended in a suitable carrier, especially an aqueous solvent for the active ingredient. The active ingredient is preferably present in such formulations in a concentration of 0.5 to 20%, advantageously 0.5 to 10% particularly about 1.5% w/w.

Formulations suitable for topical administration in the mouth include lozenges comprising the active ingredient in a flavoured basis, usually sucrose and acacia or tragacanth; pastilles comprising the active ingredient in an inert basis such as gelatin and glycerine, or sucrose and acacia; and mouthwashes comprising the active ingredient in a suitable liquid carrier.

Formulations for rectal administration may be presented as a suppository with a suitable base comprising for example cocoa butter or a salicylate.

Formulations suitable for nasal administration wherein the carrier is a solid include a coarse powder having a particle size for example in the range 20 to 200 microns which is administered in the manner in which snuff is taken, i.e. by rapid inhalation through the nasal passage from a container of the powder held close up to the nose. Suitable formulations wherein the carrier is a liquid, for administration as for example a nasal spray or as nasal drops, include aqueous or oil solutions of the active ingredient.

Formulations suitable for vaginal administration may be presented as pessaries, tampons, creams, gels, pastes, foams, or spray formulations containing in addition to the active ingredient such carriers as are known in the art to be appropriate.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets of the kind previously described.

Preferred unit dosage formulations are those containing a daily dose or unit daily sub-dose, as herein above recited, or an appropriate fraction thereof, of an active ingredient.

It should be understood that in addition to the ingredients particularly mentioned above the formulations of this invention may include other agents conventional in the art having regard to the type of formulation in question, for example those suitable for oral administration may include flavouring agents.

The present invention further provides veterinary compositions comprising at least one active ingredient as above defined together with a veterinary carrier therefor.

Veterinary carriers are materials useful for the purpose of administering the composition and may be solid, liquid or gaseous materials which are otherwise inert or acceptable in the veterinary art and are compatible with the active ingredient. These veterinary compositions may be administered orally, parenterally or by any other desired route.

For oral administration the compositions can be in the form of a tablet, granule drench, paste, cachet, capsule or feed supplement. Granules may be made by the well known techniques of wet granulation, precompression or slugging. They can be administered to animals in an inert liquid vehicle so as to form a drench, or in a suspension with water or oil base. Preferably further accessory ingredients such as dispensing agent are included. These formulations preferably contain from 15 to 85% of the active ingredient.

A paste may be formulated by suspending the active ingredient in a liquid diluent. A stiffening or thickening agent may be included together with a wetting agent or a humectant if the liquid dilent is water. If an emulsion paste is needed then one or more surface active agents should desirably be included. From 25 to 80% by weight of these paste formulations may comprise the active ingredient.

In feed supplements the active ingredient is generally present in large amounts relative to the accessory ingredients, and the supplements may be added directly or after intermediate blending or dilution. Examples of accessory ingredients for such formulations include solid, orally ingestible carriers such as corn meal, soya flour, wheat shorts, soya grits, edible vegetable materials and fermentation residues. The active ingredient is usually incorporated in one or more of the accessory ingredients and intimately and uniformly dispersed by grinding, tumbling or stirring with conventional apparatus. Formulations containing 1 to 90% by weight of the active ingredient are suitable for adding to feeds.

For the treatment of herpes infections in horses, an oral or patenteral dose of from 0.1 to 250 mg per kg body weight per day, preferably from 2 to 100 mg per kg per day may be required. The dose may be split up into discrete units administered at regular intervals during the day, and repeated daily for up to 14 days or until the infection is cleared. For viral infections in other animals the dose may vary depending on the size and metabolism of the animal. The compositions may be administered in unit dosage form, such as a tablet a few times daily in the amount of 10 to 1000 mg per unit dose.

The invention will now be illustrated by reference to the following Examples.

Example 1

a) 9-[(2-Benzoyloxy-1-benzoyloxymethyl)ethoxymethyl]guanine

To a cold (0°C) solution of acetylating mixture (prepared by combining acetic anhydride (7 ml), glacial acetic acid (3 ml) and concentrated sulfuric acid (0.1 ml)) was added, with magnetic stirring, tetrabenzoylmethylene-bis-2-glycerol (1.0 g). A. T. Ness, R. M. Hann & C. S. Hudson, *J. Amer. Chem. Soc.*, Nov 1943, 2215.

The solution was stirred at temperatures of 3° to 10°C for thirty minutes, then poured into a mixture of 260 ml of ice and water. The acidic solution was extracted three times with chloroform, and the organic extracts washed with brine and dried over sodium sulfate.

The filtered chloroform solution was evaporated *in vacuo* and the residual oil purified by column chromatography on silica gel. Elution with dichloromethane yielded 2-0-(acetoxymethyl) 1,3-bis(O-benzoyl)glycerol. The proton and carbon-13 NMR spectra were consistent with the desired structure.

A mixture of 2,9-diacetylguanine (0.49 g), 2-O-(acetoxymethyl)-1,3-bis(O-benzoyl)glycerol (1.22 g), p-toluenesufonic acid (0.013 g) in dry toluene (30 ml) was refluxed with stirring for 18 hours. The solvent was removed by flash evaporation and the residue dissolved in boiling methanol (35 ml).

n-Butanol (1 ml) was added and the mixture refluxed for five minutes, to hydrolyze the 2-acetamido group. The mixture was evaporated *in vacuo* at a bath temperature of 30°C and recrystallized once from methanol and once from formamide to yield 9-[(2-benzoyloxy-1-benzoyloxymethyl)ethoxymethyl]guanine. The elemental analysis showed the product contained 0.75 mole of formamide and the NMR spectrum was consistent with the structure. TLC on silica gel with 20% methanol in chloroform gave one spot Rf = 0.49. Mpt = 220—222°C.

b) 9-[(2-Hydroxy-1-hydroxymethylethoxy)methyl]guanine

9-[2-Benzoyloxy-1-(benzoyloxymethyl)ethoxymethyl)guanine (0.6 g) is heated in a mixture of 1:1 methanol and 40% aqueous methylamine on a steam bath for one hour. The solvents are removed *in vacuo* and the residue recrystallized twice from water to give 9-[(2-hydroxy-1-hydroxymethylethoxy)-methyl]guanine as a one quarter hydrate. m.p. dec. 230°C.

Example 2

9-[(2-Hydroxy-1-hydroxymethylethoxy)methyl]guanine

A mixture of 1,4-dichlorobutane-2,3-diol (111.8 g), paraformaldehyde (42.2 g) and boron trifluoride-etherate (22 ml) in dry acetonitrile (460 ml) was refluxed with stirring until the solids had dissolved. Molecular sieves were added and the reaction.mixture was refluxed for a further three hours. The mixture was cooled and dried by the addition of more sieves, filtered and evaporated *in vacuo*.

The oily residue was added to saturated sodium bicarbonate solution (250 ml) and extracted three times with equal volumes of purified ether. The ethereal extracts were dried over sodium sulfate, filtered and evaporated *in vacuo*.

The residual liquid was distilled with water aspirator (16 mm) pressure and the fraction boiling at 99—109°C collected to give 4,5-bis(chloromethyl)1,3-dioxolane.

A mixture of 4,5-bis(chloromethyl)1,3-dioxolane (76.5 g), and sodium benzoate (258 g) in dry dimethylformamide (1500 ml) was stirred with heating at temperature of 142°C for eighteen hours.

The mixture was cooled, filtered and the solids washed with ether. The mother liquors and washings were evaporated *in vacuo* to dryness and partitioned between dichloromethane and water, three times. The organic layers were washed with water and dried over sodium sulfate. After filtering and flash evaporation, the residue was purified by flash chromatography on silic gel in dichloromethane to give 4,5-bis(benzoyloxymethyl)-1,3-dioxolane as a thick oil. An analytical sample was obtained by allowing an aliquot of the oil to crystallize in air, mpt = 66.5—68°C.

To a chilled (0°C) solution of acetic anhydride (100 ml) and concentrated sulfuric acid (0.3 ml) was added portionwise 4,5-bis(benzoyloxymethyl)-1,3-dioxolane (114.2 g) while maintaining an internal temperature of 0—5°C. The solution was allowed to come to room temperature and then heated for three hours at steam bath temperature (internal temperature 92°C). After cooling, the solution was poured onto 600 ml of ice and water and extracted three times with purified ether. The ethereal extracts were washed once with water, once with 5% sodium bicarbonate solution and finally with brine. The ethereal extracts were dried ($Na_2SO_4$) and the clear amber solution, filtered and evaporated in vacuo to yield 2-acetoxy-3-acetoxymethoxy-1,4-butanediyl dibenzoate. An analytical sample was obtained by recrystallization from benzene-hexane. Mpt. 56—58°C.

A mixture of 2,6-dichloropurine (5.75 g) and 2-acetoxy-3-acetoxymethoxy-1,4-butanediyldibenzoate (14.1 g) was heated at 140°C under water aspirator pressure until the mixture had formed a liquid melt. After 10 minutes further heating, the reaction mixture was cooled and p-toluenesulfonic acid (150 mg) added with stirring. Heating under aspirator pressure was resumed for twenty minutes, then the reaction solution was cooled and partitioned between dichloromethane and water three times. The organic extracts were washed once with saturated sodium bicarbonate solution, twice with water and finally once with brine. After drying over sodium sulfate, and filtering, the solvent was removed by flash evaporation and the

residual yellow foam purified by column chromatography. After elution with dichloromethane to remove non-purinic by-products, elution with 50% ethyl acetatehexane yielded, on evaporation, a colorless oil with a satisfactory NMR spectrum for the desired 9-[(2-acetoxy-3-(2,6-dichloro-9H-purin-9-yl)-methoxy-1,4-butanediyl dibenzoate.

A mixture of 2-acetoxy-3-(2,6-dichloro-9H-purin-9-yl)methoxy-1,4-butanediyldibenzoate (11.5 g) and sodium azide (2.6 g) in 50 ml of 1:1 v/v ethanol-water was heated with stirring at reflux for four hours.

The heterogeneous mixture was evaporated in vacuo and the residue partitioned between ether and water. Two additional washings of the aqueous phase and evaporation of the combined, dried ethereal extracts gave 2-acetoxy-3-(2,6-diazido-9H-purin-9-yl)methoxy-1,4-butanediyldibenzoate. The NMR and IR spectra were consistent with the desired structure.

A solution of 2-acetoxy-3-(2,6-diazido-9H-purin-9-yl)methoxy-1,4-butanediyldibenzoate (5.0 g) in tetrahydrofuran (200 ml) and methanol (20 ml) containing 190 mg of 5% palladium on charcoal catalyst was shaken under 3,4 bar (50 psi) of hydrogen at room temperature for three days. The mixture was filtered through a pad of Celite® and the solution evaporated in vacuo. The syrupy residue was recrystallized from methanol to give 2 - acetoxy - 3 - (2,6 - diamino - 9H - purin - 9 - yl)methoxy - 1,4 - butanediyldibenzoate, 2 - Acetoxy - 3 - (2,6 - diamino - 9H - purin - 9 - yl)methoxy - 1,4 - butanedilydibenzoate (2.0 g) in methanol (100 ml) and 40% aqueous methylamine (35 ml) were heated on a steam bath for one hour, then the solution evaporated *in vacuo*. The residue was triturated with ether to remove the N-methylbenzamide, and recrystallized from ethanol to yield 3-(2,6-diamino-9H-purine-9-yl)methoxy 1,2,4-butanetriol, mp. 167—9°C.

A solution of 3-(2,6-diamino-9H-purine-9-yl)methoxy-1,2,4-butanetriol (0.7 g (2.4 mM)) and sodium periodate (0.6 g (2.8 mM)) in water (50 ml) was stirred at room temperature for 3.5 hours. A precipitate formed within 5 minutes of combining the two reactants.

The mixture was chilled, filtered and washed with water yielding 2-[(2-amino-1,6-dihydro-6-oxo-9H-purin-9-yl)methoxy]-3-hydroxypropanal. A mass spectrum of this product gave m/e $M^+$) = 253.

To a suspension of the aldehyde derivative (150 mg (0.592 mM)) in water (20 ml) was added in portions, sodium borohydride (18 mg (0.47 mM)). The solution was stirred at room temperature overnight, chilled and the pH adjusted to 6 with 4N aqueous hydrochloric acid. The resulting precipitate was filtered to give 9-[(2-hydroxy-1-hydroxymethylethoxy)methyl]guanine. m.p. 248—250°C.

## Example 3

9-[(2-Hydroxy-1-hydroxymethylethoxy)methyl]guanine

To a solution of 2,6-diamino-9-[(2-hydroxy-1-hydroxymethylethoxy)methyl/purine (0.5 g) in double distilled water (10 ml) is added approximately 600 ul of calf intestinal mucosa adenosine deaminase suspension in ammonium sulphate (Sigma). The mixture is incubated at 37°C and monitored by u.v. until the reaction was complete (26 days). At intervals, the reaction mixture is evaporated *in vacuo* at room temperature and redissolved in water to remove the ammonia formed and thus keep the pH of the solution between 7—7.5.

The solution is evaporated *in vacuo* and the residue is recrystallised once from methanol and twice from water to yield 9-[(2-hydroxy-1-hydroxymethylethoxy)methyl]guanine as a one quarter hydrate m.p. dec. 230°C.

## Example 4

9-[(2-hydroxy-1-hydroxymethylethoxy)methyl]-2,6-diamino purine

a) 9-[(2-benzoyloxy-1-benzoyloxymethylethoxy)methyl]-2,6-dichloropurine

A mixture of 1.16 g (6 mM) of 2,6 dichloropurine and 2.7 g (7.3 mM) of 2-O-(acetoxymethyl)-1,3-bis-(O-benzyl)glycerol was heated with magnetic stirring under water aspirator pressure at an oil bath temperature of 155°C for approximately 25 minutes. The resultant clear yellow liquid was cooled and the thick yellow glass which formed was dissolved in benzene and purified by flash chromatography on a silica gel column (diam = 6 cm). Initial elution with 1:1 benzene-dichloromethane, and dichlormethane alone, removed by-products and the desired 9-[(2-benzoyloxy-1-benzoyloxymethylethoxy)methyl]-2,6 dichloropurine was obtained by elution with 1:1 dichloromethane-ether (1.77 g). An additional crop of the 9- isomer contaminated with 7- isomer was eluted using 100% purified ether.

The H'—NMR spectrum and TLC (silica gel in dichloromethane) showed a slight amount of impurity in the main batch which is a white-yellow foam.

b) 9-[(2-Benzoyloxy-1-benzoyloxymethylethoxy)methyl]2,6-diazidopurine

A heterogeneous mixture of 1.77 g (3.53 mM) of 9-[(2-benzoyloxy-1-benzoyloxymethyl)ethoxy)methyl]-2,6-dichloropurine and 0.45 g (6.88 mM) of sodium azide in 10 ml of 1:1 v/v water-ethanol was refluxed with magnetic stirring for three hours. The solvents were removed by flash evaporation and the residual oil triturated with water to remove the sodium chloride. The oil was taken up in hot alcohol and cooled to yield, after filtration, a white solid with a pinkish tinge after drying. Mp. = 144—5°C. An additional crop was obtained from the mother liquor by evaporation and trituration with ethanol to give material sufficient purity to combine with the initial precipitate for the next step. NMR and u.v. spectra were consistent with the postulated structure.

Qual. u.v. peaks: pH 1.0 max = 240, 305, 275 (shdr)
pH 13.0 max = 300, 270 (shdr)

c) 9-[(2-Hydroxy-1-hydroxymethylethoxy)methyl]-2,6-diaminopurine

A mixture of 1.68 g (3.26 mM) of the product of stage b) in 120 ml of 1:1 v/v tetrahydrofuran-methanol was shaken with 118 mg of 5% palladium on charcoal catalyst under an initial pressure of 3,4 bar (50 psi) $H_2$ for 72 hours at room temperature. The mixture was filtrated through a pad of Celite® and the solvents removed by flash evaporation. The residual oil taken up in hot benzene and hexane which caused solidification to occur. After chilling, the solid was filtered to yield 9-[(2-benzoyloxy-1-benzoyloxymethylethoxy)methyl]-2,6,diaminopurine, which gave a satisfactory NMR (H) spectrum. The solid was dissolved in a minimum amount of methanol and stirred with an equal volume of 40% aqueous methylamine at room temperature until TLC (silica gel in 40% methanol-dichloromethane) showed complete hydrolysis had occurred. The solution was evaporated *in vacuo* and the residue recrystallised from ethanol-acetone to yield 9-[(2-hydroxy-1-hydroxymethylethoxy)methyl]-2,6-diaminopurine which gave satisfactory H′—NMR spectrum and elemental analysis. Mp. = 182—4°C.

Example 5

2,6-Diamino-9-[(2-hydroxy-1-hydroxymethylethoxy)methyl]-9H-purine

A mixture of 33.6 g (0.2 M) of 2,6-diminopurine hydrate in 400 ml of xylene was refluxed with water separation in a Dean Stark trap. Acetic anhydride, 63.84 g (0.625 M) was added and the reaction mixture was heated to distill off a mixture of acetic acid and xylene (head temperature 115°C). The temperature was raised to 125°C at the end of a four hour reaction time. To the mixture was added 0.95 g (0.005 M) of para toluenesulfonic acid hydrate and 2-O-(acetoxymethyl)-1,3-bis(O-acetyl)glycerol, 74.47 (0.3 M) and the reaction mixture heated to distill off a mixture of xylene and acetic acid at 125°C. The distillation temperature was raised to 135°C at the end of a three hour reaction time. The reaction mixture was cooled, and the crude product was obtained by flash evaporation and trituration of the residue with acetone. Recrystallization from ethanol gave the analytically pure intermediate 2,6-diacetamido 9-[(2-acetoxy-1-acetoxymethylethoxy)methyl]purine. The solid was dissolved in methanol and heated with an equal volume of 40% aqueous methylamine for 15 minutes on a steam bath. Flash-evaporation and recrystallization of the residue from ethanol gave 2,6-diamino-9-[(2-hydroxy-1-hydroxymethylethoxy)-methyl]-9H-purine.

Example 6

a) 9-[(2-hydroxy-1-hydroxymethylethoxy)methyl]-2-chloroadenine

A solution of 2.0 g (4 mM) 9-[(2-benzoyloxy-(1-benzoylkoxymethylethoxy)methyl]-2,6-dichloropurine in 65 ml of saturated methanolic ammonia was heated in a bomb at 85°C for 20 hrs. The solution was evaporated in vacuo and recrystallized twice from ethanol to yield analytically pure 9-[(2-hydroxy-1-hydroxymethylethoxy)methyl]-2-chloroadenine.

b) 9-[(2-Hydroxy-1-hydroxymethylethoxy)methyl]-2-chloro-6-hydroxypurine

To a solution of 500 mg (1.83 mM) of 9-[(2-hydroxy-1-hydroxymethylethoxy)methyl]2-chloroadenine in 10 m of glacial acetic acid was added 0.63 g (9.15 mM) of sodium nitrite in portions over a period of 45 mins. The reaction was stirred at room temperature for four hours then evaporated *in vacuo* to dryness. The residue was recrystallized once from water and once from ethanol to yield analytically pure [(-2-hydroxy-1-hydroxymethylethoxy)methyl]-2-chloro-6-hydroxypurine.

c) 9-[(2-hydroxy-1-hydroxymethylethoxy)methyl]guanine

A solution of 0.274 g (1 mM) of 9-[(2-hydroxy-1-hydroxymethylethoxy)methyl]-2-chloro-6-hydroxypurine in 60 ml of saturated methanolic ammonia was heated at 120°C in a bomb for 25 hrs. The cooled solution was evaporated *in vacuo* and recrystallized twice from water to yield analytically pure 9-[(2-hydroxy-1-hydroxymethylethoxy)methyl]guanine. Mp. 220—222°C.

Example 7

a) 5-Benzyloxy-1,3-dioxan-2-one

A solution of 182 g (1 M) of 2-O-benzylglycerol (Carbohydrate Research *91* (1981) 85—88 G. Chittenden), 128 ml of 2,6-lutidine (1.1 M) and 550 ml (1.1 M) of 20% phosgene in toluene is stirred at room temperature for two days. The solvent was removed *in vacuo* and the residue purified by factional distillation to give 157 g (75%) of 5-benzyloxy-1,3-dioxan-2-one.

b) 5-Hydroxy-1,3-dioxan-2-one

A solution of 177 g (0.85 M) of 5-benzyloxy-1,3-dioxan-2-one in 100 ml of 1.1 tetrahydrofuran-ethanol is shaken with 16 g of 5% palladium on carbon at atmospheric pressure until the theoretical amount of hydrogen is consumed. The catalyst is filtered off, the filtrate passed through a pad of Celite® and evaporated *in vacuo* to yield 94.5 g (80%) of 5-hydroxy-1,3-dioxan-2-one.

c) 5,5-Methylenedioxybis(1,3-dioxan-2-one)

A mixture of 59 g (0.5 M) of 5-hydroxy-1,3-dioxan-2-one, 45 g (0.5 M) of paraformaldehyde and 24 ml of boron trifluoride etherate in 80 ml of dry tetrahydrofuran is stirred at room temperature with 30 g of 3A molecular series for 18 hrs.

The mixture is filtered and the filtrate evaporated *in vacuo*. The residual oil is dissolved in ether and extracted twice with 5% aqueous sodium bicarbonate and twice with water. The ethereal extracts are dried over sodium sulfate, filtered and evaporated to yield 5,5-methylenedioxybis(1,3-dioxan-2-one).

d) (2-Oxo-1,3-dioxan-5-yl)oxymethylacetate

To a chilled (0°C) solution of 100 ml of acetic anhydride and 0.3 ml of concentrated sulfuric acid is added portionwise 100.4 g (0.33 M) of 5,5-methylenedioxybis(1,3-dioxan-2-one), while maintaining an internal temperature of 0—5°C. The solution is then stirred at room temperature for 18 hrs. The reaction solution is poured onto 600 ml of ice and water and extracted three times with an equal volume of purified ether. The extracts are dried over sodium sulfate, filtered and evaporated to give (2-oxo-1,3-dioxan-5-yl)oxymethyl acetate.

e) 2-Acetamido-1,9-dihydro-9-[(2-oxo-1,3-dioxan-5-yl)oxymethyl]-purine-6-one

A mixture of 6.53 g (27.8 mM) of diacetyl guanine, 0.217 g of para-toluene sulfonic acid monohydrate and 9.16 g (48.2 mM) of (2-oxo-1,3-dioxan-5-yl)-oxymethyl)acetate in 60 ml of dry xylene is refluxed with stirring for eighteen hours. The solvent is removed by evaporation *in vacuo* and the residue purified by column chromatography on silica gel, eluting the dried nine isomer with 10% methanol in dichloromethane. Recrystallization from methanol gives 5.4 g (60%) of 2-acetamido-1,9-dihydro-9-[(2-oxo-1,3-dioxan-5-yl)oxymethyl)-6H-purine-6-one.

f) 9-[(2-hydroxy-1-hydroxymethylethoxy)methyl]quanine

A solution of 0.63 g (1.96 mM) of 2-acetamido-1,9-dihydro-9-[(2-oxol,3-dioxan-5-yl)osxymethyl)-6H-purine-6-one in 30 ml of 40% aqueous methylamine is heated on a steam bath for ½ hr, then evaporated *in vacuo*. The residue is recrystallized from water to give 9-[(2-hydroxy-1-hydroxymethyl)ethoxymethyl]-quanine.

The following Examples 8 to 12 illustrate pharmaceutical formulations according to the invention where the active compound is a compound of formula (I) or a physiologically acceptable salt thereof, or a physiologically acceptable ester of a compound of formula (I').

Example 8

*Tablet*

| | |
|---|---|
| Active compound | 100 mg |
| Lactose | 200 mg |
| Starch | 50 mg |
| Polyvinylpyrrolidone | 5 mg |
| Magnesium stearate | 4 mg |
| | 359 mg |

Tablets were prepared from the foregoing ingredients by wet granulation followed by compression.

Example 9

*Injectable Solution*

| | |
|---|---|
| Active compound | 0.775 g |
| Sterile, pyrogen-free, pH 7 phosphate buffer to | 25 ml |

Example 10

*Ophthalmic Solution*

| | | | |
|---|---|---|---|
| | Active compound | | 1.0 g |
| | Sodium chloride, analytical grade | | 0.9 g |
| | Thiomersal | | 0.001 g |
| | Purified water | to | 100 ml |
| | pH adjusted | to | 5.5—7.5 |

Example 11

*Oil based Paste*

| | |
|---|---|
| China Clay (solid diluent) | 20.0% w/w |
| Mineral Oil* (liquid diluent) | 60.0% w/w |
| Active compound | 20.0% w/w |

The components were mixed to provide a paste of uniform consistency.

*Mineral oil is a high boiling fraction of a refined petroleum oil containing not less than 96% unsulphonatable material.

Example 12

*Feed Supplement — Pellets*

| | |
|---|---|
| Active compound | 1% |
| Cereal Base | 99% |

The two ingredients were mixed and the mixture then fed to any conventional feed stuff pelleting plant.

Example 13

In vivo activity of 9-[(2-hydroxy-1-hydroxymethylethoxy)methyl]quanine and acyclovir against equine rhinopneumonitis virus

*Method*

Male cream Syrian weanling hamsters, 40—50 g, 21—24 days old, were caged in grousp of 5 and given water and food *ad lib.*

Equine rhinopneumonitis virus (EHV-1), hamster adapted Vaccine Strain 'Pneumabort' was used; the vaccine was diluted $10^{-1}$ in tissue culture fluid and this stock stored in 1 ml aliquots in sealed glass vials at −70°C. On Day 0 hamsters to be infected received 0.2 ml stock vaccine virus, diluted further $2 \times 10^{-1}$ in phosphate buffered saline, subcutaneously in the left flank.

9-[(2-Hydroxy-1-hydroxymethylethoxy)methyl]guanine (hereinafter referred to as 759U) and acyclovir were made up as aqueous solutions in sterile distilled water in the concentrations specified below.

Experiment 1

*Intraperitoneal administration:* from Day 0 to Day 4, inclusive, hamsters received 759U or acyclovir in divided twice daily doses in aqueous solution, the concentration of 759U varying as following, namely 5 mg/ml to give a dose rate of 100 or 50 mg/kg/day, 1.2 mg/ml for 20 mg/kg/day, 0.18 mg/ml for 4 mg/kg/day and 0.036 mg/ml for 2 mg/kg/day. Untreated control hamsters received an equivalent volume of sterile distilled water.

*Results*

The results are shown in the following Table:

| No. in group | Treatment mg/kg/day intraperitoneal | Day 2 | Cumulative mortality 3 | 4 | 5 | 6 | 7 |
|---|---|---|---|---|---|---|---|
| 10 | 50/acyclovir | 0 | 1 | 2 | 5 | 6 | 6 |
| 10 | 100/acyclovir | 0 | 0 | 1* | 2* | 2* | 3* |
| 10 | 100/759U | 0 | 0 | 0 | 0 | 0 | 1* |
| 10 | 50/759U | 0 | 0 | 0 | 0 | 0 | 0 |
| 10 | 20/759U | 0 | 0 | 0 | 0 | 0 | 0 |
| 10 | 4/759U | 0 | 0 | 0 | 0 | 0 | 0 |
| 10 | 2/759U | 0 | 0 | 0 | 0 | 0 | 0 |
| 10 | untreated | 0 | 5 | 9 | 10 | 10 | 10 |

*non-virus specific deaths.

*Conclusion*

A dose rate of acyclovir at 100 mg/kg/day intraperitoneally for 5 days commencing 5 hours pre-infection, was generally necessary to prevent any virus-induced mortality. In comparison 759U at 2 mg/kg/day intraperitoneally gave complete control.

Experiment 2(a)

*Oral administration with 759U*

A stock solution of 759U was made in sterile distilled water at 0.304 mg/ml and then diluted in 3-fold steps to 0.101 and 0.034 mg/ml. Hamsters were given medicated drinking water for 5 days commencing 5 hours pre-infection. A control group was supplied with unmedicated water.

*Results*

| No. in group | Treatment mg/kg/day 759U oral | Day 2 | Cumulative mortality 3 | 4 | 5 | 6 | 7 |
|---|---|---|---|---|---|---|---|
| 10 | 39 | 0 | 0 | 0 | 0 | 0 | 0 |
| 10 | 13 | 0 | 0 | 0 | 0 | 0 | 0 |
| 10 | 3 | 0 | 1 | 1 | 1 | 1 | 1 |
| 10 | untreated | 1 | 5 | 10 | 10 | 10 | 10 |

*calculated from the mean daily intake.

Experiment 2(b)

*Oral administration with acyclovir*

Acyclovir was dissolved in sterile distilled water at 2 mg/ml with the addition of a little sodium hydroxide and warming to 42°C to facilitate solution. Hamsters were given medicated drinking water, containing acyclovir at 2 mg/ml, for 5 days commencing 5 hours pre-infection. Oral treatment resulted in a dose rate of approximately 100 mg/kg/day. A control group supplied with unmedicated water was set up.

*Results*

| No. in group | Treatment mg/kg/day acyclovir oral | Cumulative mortality Day 2 | 3 | 4 | 5 | 6 |
|---|---|---|---|---|---|---|
| 12 | 100 | 0 | 2 | 5 | 7 | 8 |
| 12 | untreated | 0 | 6 | 12 | 12 | 12 |

*Conclusion*

Acyclovir showed poor efficacy against EHV—1 when administered orally at 100 mg/kg/day for 5 days whilst 759U was completely effective orally at 13 mg/kg/day, with only limited mortality at a dose rate of 3 mg/kg/day.

Example 14

Comparative in vivo activities of 759U and its 6-amino analogue (457U) against equine rhinopneumonitis virus

*Materials and Methods*

*Hamsters*

50 male CO/CR Syrian hamsters were caged randomly in 10 groups of 5, with water and food (seed mix) *ad lib.* Hamsters were weighed on Days -3, 0, 4, and/or at death/sacrifice. Water consumption was recorded over Days −3 to 0 to 4.

*Virus and infection*

EHV—1 Pneumabort vaccine strain stored as a $10^{-1}$ dilution, was further diluted $10^{-1}$ in PBS for infection. On Day 0, hamsters in Groups 1—9 received 0.2 ml virus inoculum s/c in the left flank equivalent to about $2 \times 10^5$ PFU (plaque-forming units).

*Compounds and Medication*

457U and 759U were dissolved in drinking water at 0.075 mg/ml. Assuming an average daily intake of 134 mg/mg bodyweight, this would result in a daily dose of around 10 mg/kg. Medicated water was provided from approximately 5 hours pre-infection for 96 hours, as below.

| Group | Medication |
|---|---|
| 1—3 | 759U |
| 4—6 | 457U |
| 7—9 | Untreated/infected |
| 10 | Untreated/uninfected |

*Results*

Cumulative mortality patterns are summarised in the following Table. The drug intake was calculated from the weight of the animals and the water consumption recorded over the period of the experiment.

There was only 1 death from virus-induced hepatitis in the 15 457U treated hamsters compared with 14 deaths in 15 untreated controls and no deaths in 15 hamsters receiving 759U.

TABLE

| Group | Water conc. of 457U mg/ml | Water conc. of 759U mg/ml | Average intake mg/kg/day | Cumulative mortality (out of 15) on day 2 | 3 | 4+ |
|---|---|---|---|---|---|---|
| 1—3 | — | 0.075 | 6.6 | 0 | 0 | 0 |
| 4—6 | 0.075 | — | 6.8 | 0 | 1 | 1 |
| 7—9 | — | — | — | 0 | 9 | 14 |
| 10* | — | — | — | 0 | 0 | 0 |

*Conclusions*

Compound 457U, the diamino derivative of 759U, when administered orally in drinking water at a rate of approximately 7 mg/kg/day, almost completely prevented EHV—1 induced mortality in Syrian hamsters, while 759U completely prevented EHV—1 induced mortality.

Example 15

In vitro activity of 759U against Herpes simplex type 1

*Method*

60 mm plastic cell culture dishes were seeded with a VERO cell suspension (6 ml of $2 \times 10^5$ cells/ml) in VERO cell growth medium comprising 5% foetal calf serum, 10% Eagles minimal essential medium, 0.11% sodium bicarbonate, 0.25% .

*Crystamycin*

(50,000 units/ml sodium benzyl penicillin B.P. and 50 mg/ml streptomycyin sulphate B.P.). The dishes were gently shaken to ensure the complete dispersion of the cells and then the cultures were incubated at 37°C in an atmosphere of 5% $CO_2$/air overnight to attain confluency. The growth medium was then replaced with 2 ml of virus (Herpes simplex type 1) inoculum in phosphate buffered saline (PBS). The virus concentration was such as to induce the formation of 200—400 plaques/plate. A period of 1 hour was allowed for virus absorption at 37°C in 5% $CO_2$ air, after which time the plates were drained and overlay medium (8 ml) was added at 42°C. The overlay medium consisted of 0.6% agarose, 2% foetal calf serum, 10% Eagles's minimal essential medium, 0.11% sodium bicarbonate and 0.25% Crystamycin.

Doubling dilutions in micromolarity of active compound were prepared in the overlay and replicate cultures were fed with the maintenance medium overlay containing a range of concentrations of the compound. Untreated virus control cultures and uninfected were also prepared. The overlay was allowed to solidify at room temperature before the cultures were returned to the incubator at 37°C in 5% $CO_2$/air for 4 days. The cultures were then fixed with 10% formalin in PBS for 30 minutes to 1 hour; the overlay was removed and the cells were stained with 0.05% w/v methyl violet in 20% methanol. The resultant plaques were counted and expressed as a percentage of the plaque count of the untreated virus control cultures. These values were then plotted against the $\log_{10}$ of the compound concentration ad the $IC_{50}$ values representing the amount of the compound required to reduce the plaque count by 50% was read from the resulting dose-response line.

*Results*

| | 759U $IC_{50}\mu M$ | Acyclovir $IC_{50}\mu M$ |
|---|---|---|
| Experiment 1 | 0.09 | 0.17 |
| Experiment 2 | 0.05 | 0.075 |
| Experiment 3 | 0.036 | 0.11 |
| Mean | 0.059 | 0.118 |

*Conclusion*

759U has greater *in vitro* against Herpes simplex type 1 than acyclovir.

Example 16

In vivo activity of 759U against herpetic encephalitis

*Preparations of stock virus*

Swiss mice weighing 12—15 g were anaesthetised with ether and inoculated intracerebrally with 0.025 ml of a tissue culture preparation of type 1 herpes virus. The mice were examined daily for sign of herpes encephalitis i.e. cerebral irritation leading to paralysis, coma and death. Mice were killed when they showed early signs of cerebral irritation. The viscera of the mice were examined for signs of infection with bacteria or other agents and if no abnormalities were found the brains were removed acceptically and ground to a smooth paste. The homogenate was resuspended in 4 ml of tissue culture maintenance medium to each brain, and mixed with glycerol in the ratio 2:1. Mouse brains weigh approximately 400 mg and the preparation thus represented a dilution of $10^{-1}$.

The stocks of virus thus prepared were titrated in mice to determine the $LD_{50}$ titre by inoculating mice intracerebrally with 10-fold dilutions of the stock preparation of virus and subsequently examined twice daily for signs of infection. The $LD_{50}$ titre was calculated by the Karber method.

*Method*

Groups of 5 mice were infected intracerebrally with stock virus diluted to give a dose of around

300 × $LD_{50}$. The compound under test was administered twice daily as a suspension by the oral route at a dose of 100 mg/kg. The animals were examined twice daily and survival times were recorded to the nearest half-day. Dosing was carried out for 5 days and the total period of observation was 14 days. Survival times were converted to recip the mean survival time was determined for each group L. Bauer, D. J., *Brit. J. Exp. Path.*, 1960, *41*, 130).

| | Acyclovir | 759U | Untreated |
|---|---|---|---|
| Mean reciprocal survival time | 0.26 | 0.15 | 0.33 |

*Conclusion*

The activity of 759U observed when given orally to intracerebrally infected mice was much greater than acyclovir under the same conditions.

Example 17

Comparative in vivo activity of 759U and 457U against herpetic encephalitis

759U and 457U were tested *in vivo* against herpetic encephalitis in mice in comparison with acyclovir and its 6-amino analogue, 2-amino-9-(2-hydroxy-methoxymethyl)adenine (134U).

*Materials and Methods*

Mice

CD.1 mice weighing 16—20 gm were obtained from Charles River, Manston, Kent, U.K. The animals were housed in groups of approximately 10 and were given food and water ad lib.

*Antiviral compounds*

The compounds namely acyclovir, 134U, 759U and 457U, were prepared as suspensions or solutions in sterile water at a concentration of 20 mg/ml prior to each experiment, and were stored at 4°C during the dosing period.

*Virus*

The ICI strain of herpes simplex virus type 1 was used for all experiments at a concentration of 300 $LD_{50}$ ($10^5$ pfu/ml). Dilutions from virus stocks ($10^7$ pfu/ml) were prepared in PBS 'A'.

*Test procedures*

The test procedure is analogous to that described in Example 12.

Three experiments were performed, in which three routes of compound administration were examined: i) subcutaneous, ii) oral and iii) intraperitoneal. Compounds were administered at 100 mg/ml/dose twice daily for 4½ days, commencing 2—3 hours after infection.

*Results*

The mean reciprocal survival times following oral, subcutanteous (s.c.) and intraperitoneal (i.p.) administration of the antiviral compounds are shown in the following Table.

| Treatment Group | Mean reciprocal survival time | | |
|---|---|---|---|
| | Oral | s.c. | i.p. |
| Virus controls 300 $LD_{50}$ | 0.334 | 0.344 | 0.307 |
| Acyclovir 100 mg/kg/dose | 0.237 | 0.174 | 0.236 |
| 134U 100 mg/kg/dose | 0.222 | 0.226 | 0.231 |
| 759U 100 mg/kg/dose | 0.146 | 0.084 | 0.093 |
| 457U 100 mg/kg/dose | 0.155 | 0.102 | 0.077 |

Comparing the two groups of therapies, acyclovir and 134U with 759U and 457U, the latter group resulted in the greatest antiviral effect. The differences between the two groups was highly significant by any of the three routes of administration.

## Claims

1. The compound of the formula

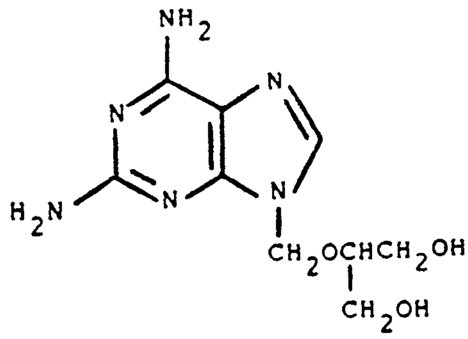

(I)

and physiologically acceptable acid addition salts thereof.

2. Physiologically acceptable esters of compounds of formula (I'):

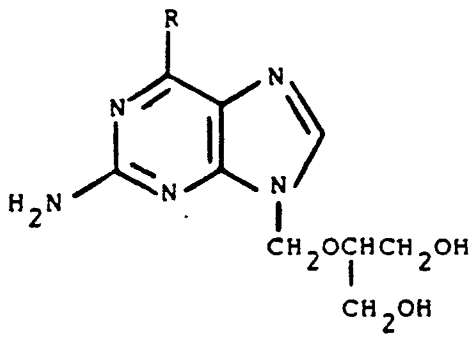

(I')

(wherein R represents a hydroxy or amino group and wherein the ester group is a formyloxy, $C_{1-16}$ alkanoyloxy, or an aralkanoyloxy or aroyl ester grouping optionally substituted by one or more halogen atoms or amino, nitrile or sulphamido groups.

3. Physiologically acceptable esters as claimed in claim 2 wherein the ester group is an acetoxy or benzoyloxy ester grouping.

4. A process for preparing compounds of formula (I') (as defined in claim 2) and physiologically acceptable salts and esters thereof (as defined in claim 2) characterized in that

(a) a compound of formula (II)

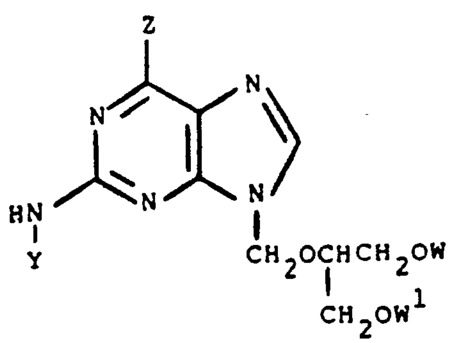

(II)

(wherein W and $W^1$ each represent a hydrogen atom or a blocking group, Y is a hydrogen atom or a blocking group and Z is a group of formula —OY or —NHY, wherein Y is as defined above with the provisos that (a) when Z represents a hydroxy group and Y represents an acyl blocking group, W and $W^1$ each represent a blocking group other than an aralkyl group, (b) when Z represents a hydroxy group and W and $W^1$ each represent a hydrogen atom, Y represents hydrogen or blocking group other than an acyl blocking group, and (c) at least one of W, $W^1$ and Y represents a blocking group), is deblocked to form a compound of formula (I') or a salt or ester thereof;

(b) a compound of formula (III)

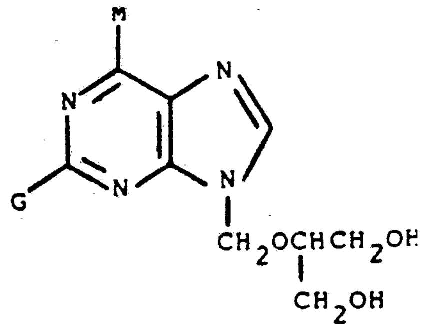

(III)

(wherein M is a 6-hydroxy or amino group and G is an atom or other group than an acyl group that can be replaced by or converted to an amino group, or G is a 2-amino group and M is an atom or group that can be replaced by or converted to an amino or hydroxy group) or a salt or ester thereof is converted to a compound of formula (I') or a salt or ester thereof;

(c) a compound of formula (IV)

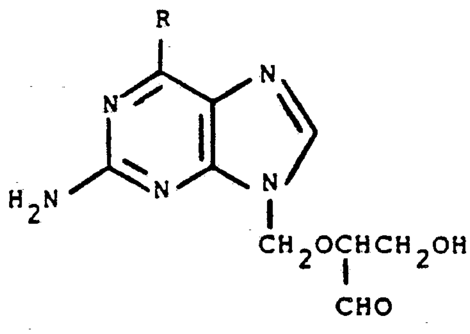

(IV)

(wherein R is as defined in claim 2 or a salt or ester thereof is reduced;

(d) a compound of the formula

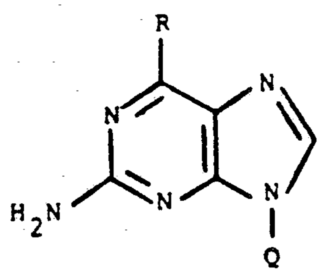

(V)

(wherein R is as defined in claim 2 and Q is a leaving group or atom) is reacted with a compound of formula

$ACH_2OCHCH_2OH$
|
$CH_2OH$ (VI)

(wherein A is leaving group or atom);

(e) hydrolysis of a compound of formula

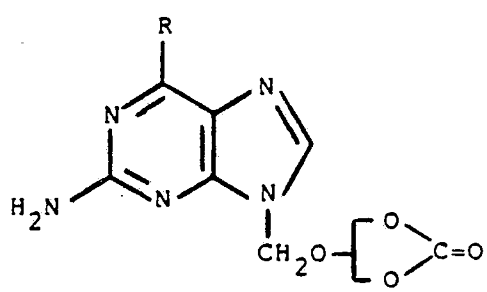

(VII)

(wherein R is as defined above); and optionally effecting one or more of the following conversions, in any desired sequence:

(i) where the resulting product is a base, converting the said base into a physiologically acceptable acid addition salt thereof;

(ii) where the resulting product is an acid addition salt, converting the said salt into the parent base;

(iii) where the resulting product is a compound of formula (I') or a salt thereof converting the said compound or salt thereof into a physiologically acceptable ester of the said compound or salt; and/or

(iv) where the resulting product is an ester of a compound of formula (I), converting the said ester into the parent compound of formula (I') or a physiological acceptable salt thereof.

5. Pharmaceutical or veterinary formulations comprising, as active ingredient, at least the compound of formula (I) (as defined in claim 1) or a salt thereof or a physiologically acceptable ester of a compound of formula (I') (as defined in claim 2) together with one or more carriers therefor.

6. Formulations as claimed in claim 5 adapted for oral administration.

7. Formulations as claimed in claim 6 in the form of tablets or capsules.

8. Formulations as claimed in claim 5 adapted for parenteral administration.

9. Formulations as claimed in claim 5 adapted for topical administration.

10. A compound of formula (I) (as defined in claim 1) and physiologically acceptable salts thereof and physiologically acceptable esters of a compound of formula (I') as defined in claim 2 for use in the treatment or prophylaxis of a viral disease in an animal.

11. A compound of formula (I) as claimed in claim 10 wherein said compound is 9-[(2-hydroxy-1-hydroxymethylethoxy)methyl]-2,6-diaminopurine.

12. A compound as claimed in claim 10 or 11 for the use in treatment or prophylaxis of a herpes viral disease.

13. A compound as claimed in claims 10 or 11 for use in the treatment of a viral disease in a human or other animal.

**Patentansprüche**

1. Verbindung der Formel

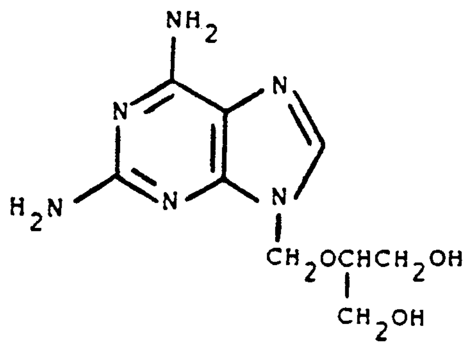

(I)

und physiologisch annehmbare Säureadditionssalze davon.

2. Physiologisch annehmbare Ester von Verbindungen der Formel (I'):

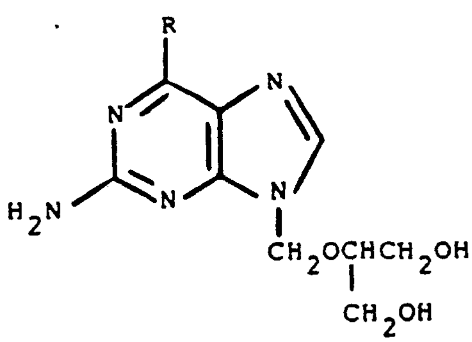

(I')

worin R eine Hydroxy- oder Aminogruppe bedeutet und worin die Estergruppe eine Formyloxy, $C_{1-16}$ Alkanoyloxy- oder eine Aralkanoyloxy- oder eine Aroylestergruppe ist, die gegebenenfalls durch eines oder mehrere Halogenatome oder Amino-, Nitril- oder Sulfamidogruppen substituiert ist.

3. Physiologisch annehmbare Ester nach Anspruch 2, worin die Estergruppe eine Acetoxy- oder Benzoyloxyestergruppe ist.

4. Verfahren zur Herstellung von Verbindungen der Formal (Í), (wie in Anspruch 2 definiert), und

physiologisch annehmbarer Salze und Ester davon wie in Anspruch 2 definiert, dadurch gekennzeichnet, daß man

(a) eine Verbindung der Formel (II)

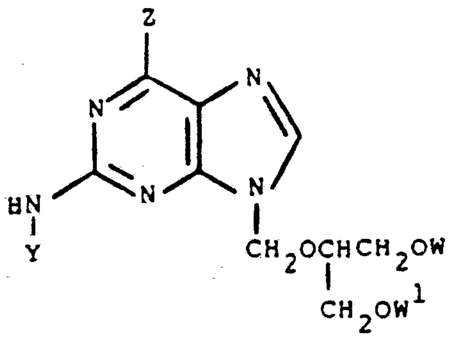

(II)

(worin W und W[1] jeweils ein Wasserstoffatom oder eine Blockierungsgruppe bedeuten, Y ein Wasserstoffatom oder eine Blockierungsgruppe ist und Z eine Gruppe der Formel —OY oder -NHY bedeutet, worin Y die oben angegebene Bedeutung besitzt, mit den Maßgaben, daß (a) wenn Z eine Hydroxygruppe und Y eine Acylblockierungsgruppe bedeutet, W und W[1] jeweils eine Blockierungsgruppe verschieden von einer Aralkylgruppe bedeuten, (b) wenn Z eine Hydroxygruppe und W und W[1] jeweils ein Wasserstoffatom bedeuten, Y Wasserstoff oder eine Blockierungsgruppe, verschieden von einer Acylblockierungsgruppe bedeutet, und (C) mindestens von W und W[1] und Y bedeutet eine Blockierungsgruppe), zur Bildung einer Verbindung der Formel (I') oder eines Salzes oder Esters davon entblockt;

b) eine Verbindung der Formel (III)

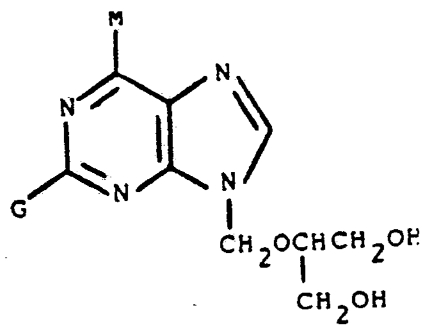

(III)

(worin M eine 6-Hydroxy- oder Aminogruppe ist und G ein Atom oder eine andere Gruppe als eine Acylgruppe ist, die durch eine Aminogruppe ersetzt oder in eine Aminogruppe überführt werden kann, oder G ist eine 2-Aminogruppe und M ist ein Atom oder eine Gruppe, die durch eine Amino- oder Hydroxygruppe ersetzt, oder in eine Amino-der Hydroxygruppe überführt werden kann), oder ein Salz oder einen davon in eine Verbindung der Formerl I' oder ein Salz oder Ester davon überführt;

c) eine Verbindung der Formel (IV)

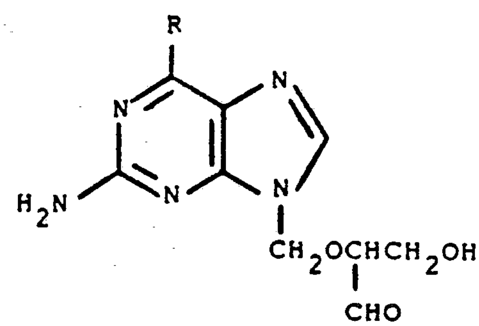

(IV)

worin R die in Anspruch 2 gegebene Bedeutung aufweist oder ein Salz oder einen Ester davon reduziert;

d) eine Verbindung der Formel (V)

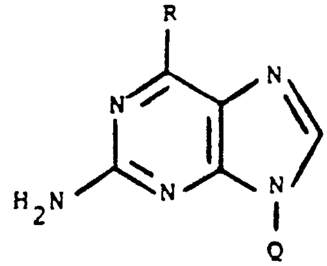

(V)

(worin R die Anspruch 2 gegebene Bedeutung aufweist und Q eine austretende Gruppe oder Atom bedeutet) mit einer Verbindung der Formel VI

$$ACH_2OCHCH_2OH$$
$$CH_2OH$$

(worin A eine austretende Gruppe oder Atom bedeutet) umsetzt;

e) eine Verbindung der Formel (VI)

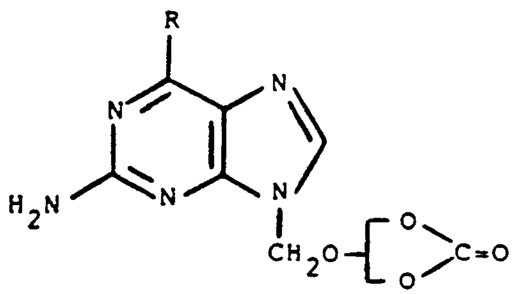

(VI)

(worin R die vorhin gegebene Bedeutung aufweist), hydrolisiert und gegebenenfalls eine oder mehrere der folgenden Unwandlungen in jeder gewünschten Reihenfilge durchführt:

(i) Wenn das erhaltene Produkt eine Base ist, Überführung der Base in ein physiologisch annehmbares Säureadditionssalz davon;

(ii) Wenn das erhaltene Produkt ein Säureadditionssalz ist, Überführung dieses Salzes in die Stammbase;

(iii) Wenn das erhaltene Produkt eine Verbindung der Formel (I') oder ein Salz davon ist, Überführung dieser Verbindung oder des Salzes davon in einen physiologisch annehmbaren Ester dieser Verbindung oder in das Salz; und/oder

(iv) Wenn das erhaltene Produkt ein Ester einer Verbindung der Formel (I) ist, Überführung des Esters in die Stammverbindung der Formel (I') oder ein physiologisch annehmbares Salz davon.

5. Pharmazeutische oder veterinärmedizinische Formulierungen, enthaltend als aktiven Bestandteil mindestens eine Verbindung der Formel (I) (wie in Anspruch 1 definiert) oder ein Salz davon oder einen physiologisch annehmbaren Ester, einer Verbindung der Formel (I'), (wie in Anspruch 2 definiert), zusammen mit einem oder mehreren Trägerstoffen dafür.

6. Formulierungen nach Anspruch 5 angepaßt für die orale Verabreichung.

7. Formulierungen nach Anspruch 6 in Form von Tabletten oder Kapseln.

8. Formulierungen nach Anspruch 5, angepaßt für die parenterale Verabreichung.

9. Formulierungen nach Anspruch 5, angepaßt für die topische Verabreichung.

10. Verbindung der Formel (I), (wie in Anspruch 1 definiert), und physiologisch annehmbare Salze davon und physiologisch annehmbare Ester einer Verbindung der Formel (I'), (wie in Anspruch 2), definiert zur Verwendung in der Behandlung oder Prophylaxe einer viralen Erkrankung bei einem Tier.

11. Verbindung der Formel (I) nach Anspruch 10, worin die Verbindung 9-[(2-Hydroxy-1-hydroxmethylethoxy)methyl]-2,6-diaminopurin ist.

12. Verbindung nach Anspruch 10 oder 11 zur Verwendung in der Behandlung oder Prophylaxe einer Herpes Viruserkrankung.

13. Verbindung nach Anspruch 10 oder 11 zur Verwendung in der Behandlung einer Viruserkrankung bei einem Menschen oder einem anderen Tier.

**Revendications**

1. Composé de formule

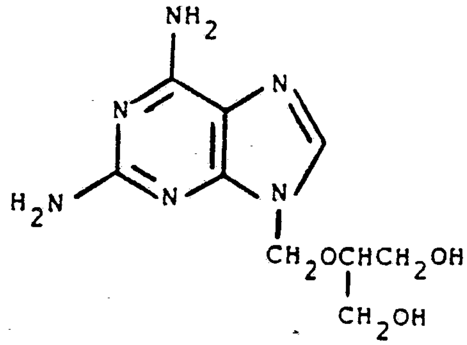

(I)

et ses sels d'addition d'acides physiologiquement acceptables.

2. Esters physiologiquement acceptables de composés de formule (I')

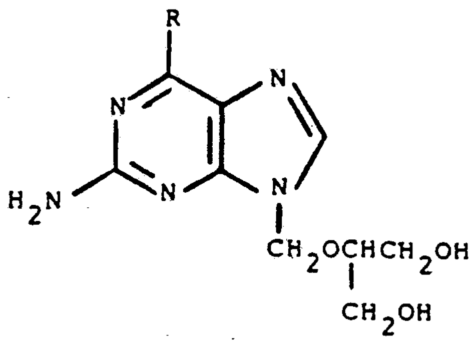

(I')

(où R représente un radical hydroxyle ou amino) et où le radical ester est un groupement ester formyloxy ou $C_{1-16}$ alcanoyloxy ou bien aralcanoyloxy ou aroyle éventuellement substitué par un ou plusieurs atomes d'halogène ou radicaux amino, nitrile ou sulfamido.

3. Esters physiologiquement acceptables suivant la revendication 2, dans lesquels le radical ester est un groupement ester acétoxy ou benzoyloxy.

4. Procédé de préparation de composés de formule (I') (tels que définis dans la revendication 2) et de leurs sels et esters physiologiquement acceptables (tels que définis dans la revendication 2), caractérisé en ce que

(a) un composé de formule (II)

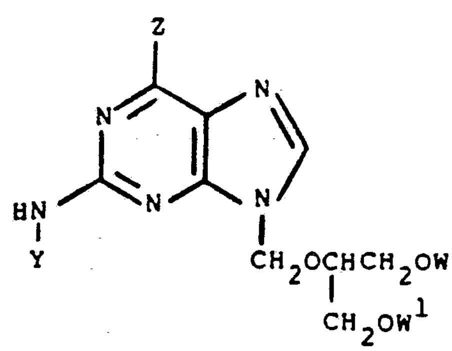

(II)

(où W und $W^1$ représentent chacun un atome d'hydrogène ou un fardical de blocage, Y représente un atome d'hydrogène ou un radical de blocage et Z représente un radical de formule —OY ou —NHY, où Y est tel que défini ci-dessus, avec les restrictions que (a) lorsque Z représente un radical hydroxyle et Y représente un radical acyle de blocage, W et $W^1$ représentent chacun un radical de blocage autre qu'un radical aralcoyle, (b) lorsque Z représente un radical hydroxyle et W et $W^1$ représentent chacun un atome d'hydrogène, Y représente un atome d'hydrogene ou un radical de blocage autre qu'un radical acyle de blocage et (c) au moins l'un d'entre W, $W^1$, et Y représente un radical de blocage),

et débloqué pour la formation d'un composé de formule (I') ou d'un sel ou ester de celui-ci;

(b) un composé de formule (III)

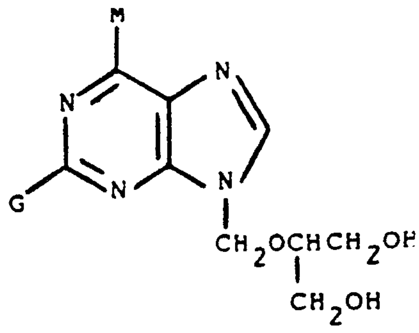

(III)

(où M représente un radical 6-hydroxyle ou -amino et

G représente un atome ou radical autre qu'un radical acyle qui peut être remplacé par ou converti en un radical amino, ou bien G représente un radical 2-amino et M represénte un atome qui peut être remplacé par ou converti et un radical amino ou hydroxyle)

ou un sel ou ester de celui-ci est converti en un composé de formule (I') ou en un sel ou ester de celui-ci;

(c) un composé de formule (IV)

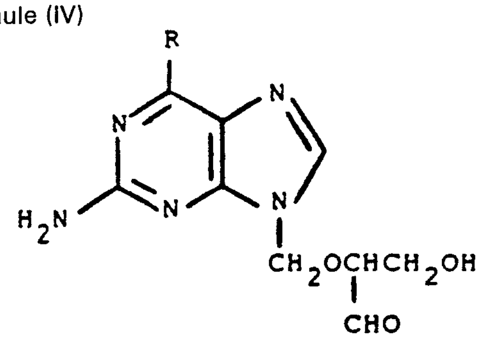

(IV)

(où R est tel que défini dans la revendication 2) ou un sel ou ester de celui-ci est réduit;

(d) un composé de formule

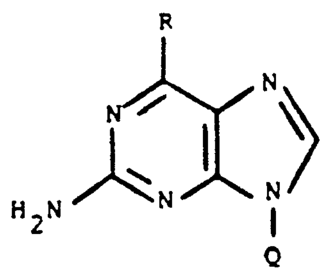

(V)

(où R est tel que défini dans la revendication 2 et Q représente un radical ou atome partant) est mis à réagir avec un composé de formule

$$ACH_2OCHCH_2OH$$
$$|$$
$$CH_2OH$$

(VI)

(où A représente un radical ou atome partant);

(e) un composé de formule

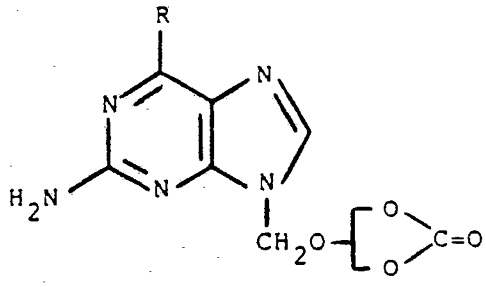

(VII)

(où R est tel que défini ci-dessus); est hydrolysé et une ou plusieurs des conversions ci-après sont facultativement effectuées en toute succession souhaitées;

(i) lorsque le produit résultant est une base, la conversion de la base en un sel d'addition d'acide physiologiquement acceptable de celle-ci;

(ii) lorsque le produit résultant est un sel d'addition d'acide, la conversion de ce sel en la base à laquelle il est apparenté.

(iii) lorsque le produit résultant est un composé de formule (I') ou un sel de celui-ci, la conversion de ce composé ou de son sel en un ester physiologiquement acceptable de ce composé ou sel, et/ou

(iv) lorsque le produit résultant est un ester d'un composé de formule (I) la conversion de cet ester en le composé de formule (I') auquel il est apparenté ou en un sel physiologiquement acceptable de celui-ci.

5. Compositions pharmaceutiques ou vétérinaires comprenant comme constituant actif au moins le composé de formule (I) (tel que défini dans la revendication 1) ou un sel de celui-ci ou un ester physiologiquement acceptable d'un composé de formule (I') (tel que défini dans la revendication 2), conjointement avec un ou plusiers excipients.

6. Compositions suivant la revendication 5, propres à l'administration par voie orale.

7. Compositions suivant la revendication 6, sous la forme de comprimés ou de gélules.

8. Compositions suivant la revendication 5, propres à l'administration parentérale.

9. Compositions suivant la revendication 5, propres à l'administration topique.

10. Composé de formule (I) (tel que défini dans la revendication 1) et sels physiologiquement acceptables de celui-ci et esters physiologiquement acceptables d'un composé de formule (I') (tels que définis dans la revendication 2; à utiliser pour le traitement ou la prophylaxie d'une affection virale chez un animal.

11. Composé de formule (I) suivant la revendication 10, qui est la 9-[(2-hydroxy-1-hydroxyméthyléthoxy)méthyl]-2,6-diaminopurine.

12. Composé suivant la revendication 10 ou 11, à utiliser pour le traitement ou la prophylaxie d'une affection virale herpétique.

13. Composé suivant la revendication 10 ou 11, à utiliser pour le traitement d'une affection virale chez l'être humain ou un autre animal.